(19) Europäisches Patentamt
European Patent Office
Office européen des brevets

(11) **EP 3 832 663 A1**

(12) **EUROPEAN PATENT APPLICATION**
published in accordance with Art. 153(4) EPC

(43) Date of publication:
**09.06.2021 Bulletin 2021/23**

(51) Int Cl.:
**G16H 50/20** (2018.01)   **A61B 5/00** (2006.01)

(21) Application number: **19843825.1**

(22) Date of filing: **31.07.2019**

(86) International application number:
**PCT/JP2019/030091**

(87) International publication number:
**WO 2020/027228 (06.02.2020 Gazette 2020/06)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME
KH MA MD TN**

(30) Priority: **31.07.2018 JP 2018144549**

(71) Applicant: **Lily Medtech Inc.
Tokyo 113-0033 (JP)**

(72) Inventors:
• **TOMII, Naoki
  Tokyo 113-0033 (JP)**
• **NAKAMURA, Hirofumi
  Tokyo 113-0033 (JP)**

(74) Representative: **Wallin, Nicholas James
Withers & Rogers LLP
4 More London Riverside
London SE1 2AU (GB)**

(54) **DIAGNOSTIC SUPPORT SYSTEM AND DIAGNOSTIC SUPPORT METHOD**

(57) Provided is a diagnostic support system (IS) comprising: a pre-acquired data storage unit (3) which stores, as comparison images, a plurality of images of internal information of a subject that were acquired in advance, wherein the plurality of comparison images are provided with image data, label information indicating biological tissue information and shape information, and feature value information enabling identification of the degree of similarity between the plurality of comparison images; a calculation unit (13) which inputs, into a pre-trained model that was trained on the plurality of comparison images, image data pertaining to an examination image acquired from a subject serving as the target of examination, and calculates feature value information for the examination image; and a display unit (16) which, on the basis of the feature value information for the plurality of comparison images and the feature value information for the examination image, displays the similarity between the examination image and the plurality of comparison images. As a result, it is possible to enhance the accuracy of a diagnosis by displaying the information used as the basis for the determination by a diagnostic support device.

FIG. 1

EP 3 832 663 A1

**Description**

TECHNICAL FIELD

The present invention relates to a diagnostic support system and a diagnostic support method pertaining to display of basis for a determination result by image diagnostic support using a computer.

BACKGROUND ART

A medical imaging system using CT, MRI, ultrasound, or the like is not accompanied by surgery in which a living body is directly cut and observed. Thus, the medical imaging system has widely been used, as a technique of imaging internal information of a subject, in the medical field.

A doctor who uses an acquired image to determine presence or absence of a tumor, or the like needs to have substantial experience to accurately interpret the image. Meanwhile, due to advancement of the imaging technique, the number of the images per subject is increased. As a result, a user has to effectively identify the image suggesting necessity of the determination from a large number of the images, which increases a burden associated with the interpretation of the images. For example, in a case of breast cancer screening, a probability of a cancer patient in a subject group is approximately 0.5%. Thus, the extremely small number of the images suggesting breast cancer have to be carefully found from the large number of the images, which significantly increases the burden associated with the interpretation of the images.

As a device for supporting the interpretation of the images, a diagnostic support device has been developed. The diagnostic support device acquires an examination image from the medical imaging system, detects an abnormal region, such as the tumor, in the image through image processing or machine learning processing, and presents the detected abnormal region to a doctor or the like so as to support an image diagnosis.

For example, a case image retrieval device including a finding information output unit (see Patent Document 1), a similar image retrieval device including a feature value calculation unit, a probability calculation unit, and a degree of similarity calculation unit (see Patent Document 2), and the like have been proposed. The finding information output unit associates finding information with a similar image retrieved by a retrieval unit and outputs the finding information to a specified output device. The finding information corresponds to a feature value that contributes to retrieval of the similar image. The feature value calculation unit calculates the feature value that corresponds to a pre-registered lesion pattern. The probability calculation unit calculates a probability of existence of the lesion pattern in the retrieved image on the basis of the feature value. The degree of similarity calculation unit calculates a degree of similarity.

However, while image diagnostic support display for the doctor using machine learning displays the calculated probability of cancer as well as the similar image, basis of a displayed content is not presented.

In the case of supporting a doctor's diagnosis, even when the probability of cancer is calculated and displayed, it is difficult for the doctor to make a diagnosis without the basis therefor. In addition, in the case where the acquired probability is presented while basis for such a numerical value is not presented, a diagnostic result relies on the determination by the doctor. As a result, the numerical value itself becomes meaningless.

Furthermore, in the case where a similar case is displayed while basis for detection of the displayed similar case is not presented, accuracy of the similar case itself becomes vague for the doctor. As a result, a degree of contribution of the similar case to the diagnosis becomes low.

RELATED ART DOCUMENT

PATENT DOCUMENTS

Patent Document 1: JP 2011-118543 A
Patent Document 2: JP 2016-45662 A

SUMMARY OF THE INVENTION

PROBLEM TO BE SOLVED BY THE INVENTION

The present invention has been made in view of the above points and provides a diagnostic support system and a diagnostic support method enhancing accuracy of a diagnosis by displaying information used as basis for a determination by a diagnostic support device.

MEANS FOR SOLVING THE PROBLEM

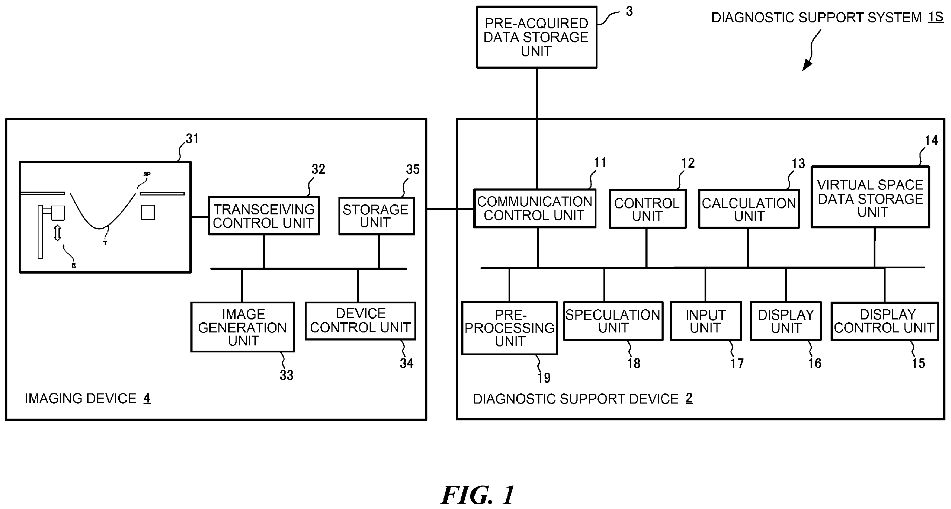

To achieve the above object, a diagnostic support system 1S according to the first aspect of the present invention includes, as shown in FIG. 1, for example, a pre-acquired data storage unit 3 for storing, as comparison images, two or more images of internal information of a subject, the two or more comparison images being acquired in advance and each comparison image including image data, label information indicating biological tissue information or shape information, and feature value information with which a degree of similarity among the two or more comparison images can be identified; a calculation unit 13 for inputting image data of an examination image acquired from a subject as an examination target to a learned (trained) model that has learned the two or more comparison images, so as to calculate feature value information of the examination image; and a display unit 16 for displaying the similarity between the examination image and each of the two or more comparison images on the basis of the feature value information of the two or more comparison images and the feature value information of the examination image.

With this configuration, the display unit can display the similarity between the examination image and each of the plural comparison images. Thus, compared to a case where a probability of cancer or a similar case is only displayed, beneficial information that can support a doctor to make a diagnosis can additionally be provided.

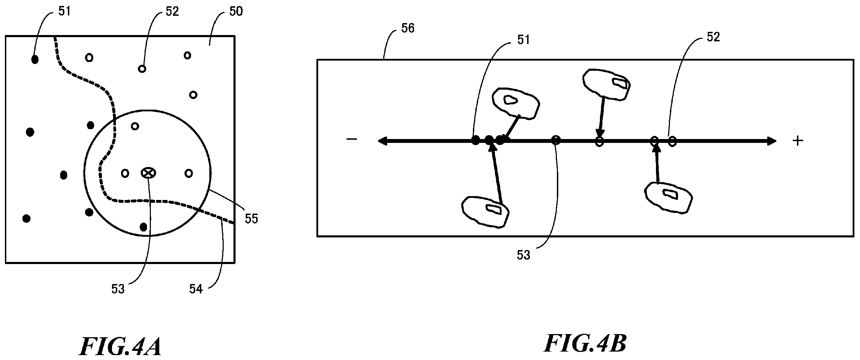

As for the diagnostic support system 1S according to the second aspect of the present invention, as shown in FIGs. 1 and 4, for example, in the diagnostic support system 1S according to the first aspect, the feature value information is information including a multidimensional parameter, and the system further includes a control unit 12 for identifying, as a value indicating the similarity, a distance L between the examination image 53 and each of the two or more comparison images 51, 52 on a feature value space 50 that has the multidimensional parameter constituting the feature value information as a coordinate.

With this configuration, the similarity can be expressed by a numerical value. Thus, the similarity can reliably be identified.

As for the diagnostic support system 1S according to the third aspect of the present invention, as shown in FIGs. 1, 4 and 7, for example, in the diagnostic support system 1S according to the first aspect, the feature value information is information including a multidimensional parameter, the system further includes a virtual space data storage unit 14 for generating a first virtual space by using the feature value information, the first virtual space including a displayable dimensional number and being used to visually recognize the similarity, and in addition to the two or more comparison images, the display unit 16 displays a first virtual space image, the examination image being plotted on the first virtual space in the first virtual space image.

With this configuration, as a method for displaying the similarity between the examination image and each of the plural comparison images, it is possible to display the virtual space image in which these images are plotted on the space. Thus, the similarity therebetween can be understood at first glance.

As for the diagnostic support system 1S according to the fourth aspect of the present invention, as shown in FIG. 1, for example, in the diagnostic support system 1S according to the first aspect, the feature value information is information including linguistic expression corresponding to the two or more comparison images and the examination image, the system further includes a virtual space data storage unit 14 for generating a second virtual space by using the feature value information, the second virtual space being used to visually recognize the similarity, and in addition to the two or more comparison images, the display unit 16 displays a second virtual space image, the examination image being plotted on the second virtual space in the second virtual space image.

With this configuration, as the method for displaying the similarity between the examination image and each of the plural comparison images, it is possible to display the virtual space image in which these images are plotted on the space. Thus, the similarity therebetween can be understood at first glance.

As for the diagnostic support system 1S according to the fifth aspect of the present invention, as shown in FIG. 1, for example, in the diagnostic support system 1S according to the third or fourth aspect, the similarity is identified on the basis of a distance between the examination image and each of the two or more comparison images on the virtual space image.

With this configuration, the similarity can be expressed by the numerical value. Thus, the similarity can easily be understood.

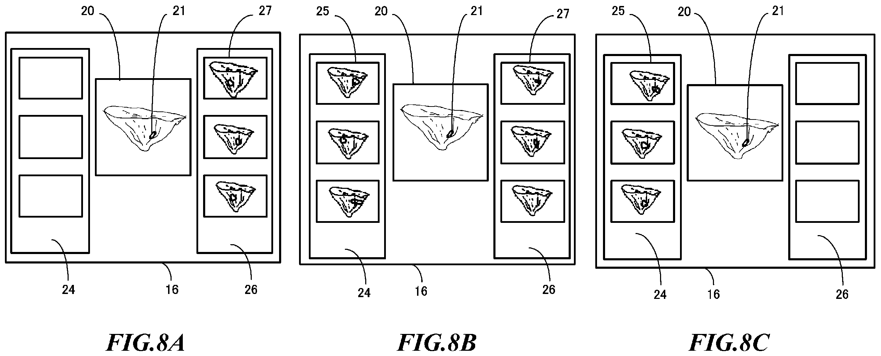

As for the diagnostic support system 1S according to the sixth aspect of the present invention, as shown in FIGs. 1 and 8, for example, in the diagnostic support system 1S according to any one of the first to fifth aspect, the display unit16 displays one or more of the two or more comparison images, the similarity of which to the examination image is high.

With this configuration, the comparison image with the high degree of similarity can be selected and then displayed. As a result, the doctor can further easily make the diagnosis.

As for the diagnostic support system 1S according to the seventh aspect of the present invention, as shown in FIG. 1, for example, in the diagnostic support system 1S according to any one of the first to sixth aspect, the display unit 16 adds a specified visual effect to display of the two or more comparison images on the basis of the label information.

With this configuration, the label information of each of the comparison images can be displayed by adding the specified visual effect. Thus, the doctor can understand a diagnosis result of the comparison images and the like at first glance when making the diagnosis.

As for the diagnostic support system 1S according to the eighth aspect of the present invention, as shown in FIG. 1, for example, in the diagnostic support system 1S according to any one of the first to seventh aspect, the system further includes a speculation unit 18 for speculating on the basis of the examination image and the two or more comparison images whether preset biological information is included in the examination image.

With this configuration, it is possible to display whether a biological image, for example, a malignant tumor or the like is included in the examination image. Thus, the doctor can easily make a determination by the diagnosis.

As for the diagnostic support system 1S according to the ninth aspect of the present invention, as shown in FIG. 1, for example, in the diagnostic support system 1S according to any one of the first to eighth aspect, the system further includes a pre-processing unit 19 for adjusting a format of the image data of the examination image to a format with which the calculation unit 13 can calculate the feature value information.

With this configuration, even in the case where the image data of the examination image is in such a data format that it is difficult for the calculation unit to calculate the feature value information as is, the data format can be adjusted. As a result, it is possible to provide the diagnostic support system that can provide the diagnostic support regardless of a type of the examination image.

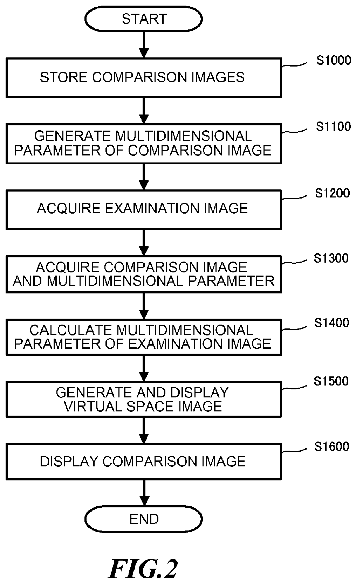

A diagnostic support method using a computer according to the tenth aspect of the present invention includes, as shown in FIG. 2, for example, storing, as comparison images, two or more images of internal information of a subject, the two or more comparison images being acquired in advance and each comparison image comprising image data and feature value information with which a degree of similarity among the two or more comparison images can be identified S1000; inputting image data of an examination image acquired from a subject as an examination target to a learned model that has learned the two or more comparison images, so as to calculate feature value information of the examination image S1400; and displaying the similarity between the examination image and each of the two or more images on the basis of the feature value information of each of the two or more images and the feature value information of the examination value S1500.

With this configuration, compared to the case where the similarity between the examination image and each of the plural comparison images is displayed, and thus only the probability of cancer or the similar case is displayed, the beneficial information for supporting the doctor to make the diagnosis can additionally be provided.

EFFECT OF INVENTION

The diagnostic support system according to the present invention can enhance accuracy of the diagnosis by displaying the information used as the basis for the determination by the diagnostic support device and can reduce a burden of image interpretation.

BRIEF DESCRIPTION OF DRAWINGS

FIG. 1 is a schematic block diagram illustrating a configuration of a diagnostic support system according to a first embodiment of the present invention.

FIG. 2 is a flowchart illustrating an outline of operation of the diagnostic support system according to the first embodiment of the present invention.

FIG. 3 includes schematic views of a display unit according to the first embodiment of the present invention.

FIG. 4 includes first schematic views of a virtual space image according to the first embodiment of the present invention.

FIG. 5 includes second schematic views of the virtual space image according to the first embodiment of the present invention.

FIG. 6 includes third schematic views of the virtual space image according to the first embodiment of the present invention.

FIG. 7 includes fourth schematic views of the virtual space image according to the first embodiment of the present invention.

FIG. 8 includes other schematic views of the display unit according to the first embodiment of the present invention.

FIG. 9 includes schematic views of a display unit according to a third embodiment of the present invention.

DETAILED DESCRIPTION OF THE INVENTION

This application is based on the Patent Application No. 2018-144549 filed on July 31, 2018 in Japan, the contents of which are hereby incorporated in its entirety by reference into the present application, as part thereof.

The present invention will become more fully understood from the detailed description given hereinbelow. Further range of application of the present invention will become clearer from the detailed description given hereinbelow. However, the detailed description and the specific embodiment are illustrated of desired embodiments of the present invention and are described only for the purpose of explanation. Various changes and modifications will be apparent to those ordinary skilled in the art on the basis of the detailed description.

The applicant has no intention to give to public any disclosed embodiment. Among the disclosed changes and modifications, those which may not literally fall within the scope of the patent claims constitute, therefore, a part of the present invention in the sense of doctrine of equivalents.

At first, a description will be made on an outline of a classification method using machine learning. First, in order to describe classification using the machine learning, a description will be made on, as a comparison target, a method for classifying data on a space spanned on N-dimensional orthonormal basis E (ei, $e_2$, ..., $e_N$). In the case where E is the orthonormal basis, data d can be expressed by using the base as in the following mathematical formula (i).

$$d = \sum_{i=1}^{N} w_i e_i \qquad \qquad \cdot \cdot \cdot \text{(i)}$$

For example, in Fourier expansion of a waveform signal using a trigonometric function as the orthonormal basis, a waveform can be decomposed using orthogonal basis E and can be classified using distribution (spectrum) of a weight value $w_i$. As a method other than the Fourier expansion, a method for calculating an eigenvector capable of classifying data the most by converting the eigenvector has been studied for a long period of time. However, due to limited expression of an eigenspace, it is difficult for a machine to determine classification of images that can be identified by a person. In recent years, an image recognition field has drawn significant attention due to improvement in accuracy of the classification, which is achieved by diversity of acquired expression using the machine learning such as deep learning. In the case where the data is projected onto a multidimensional space, in which accuracy of separation in a classification process is high, via a so-called convolution network, and a boundary between plural sets can be defined in this multidimensional space, the data can be classified. However, there is a case where it is difficult to set a boundary line such as classification of a lesion part and a normal area and classification of a lesion from the plural lesions. The characteristic of the present invention is to make a diagnostic reading doctor acknowledge, by visually displaying the vague boundary line, that his/her determination is necessary on the multidimensional space with a difficulty in setting the boundary line.

<First Embodiment

Referring to Fig. 1, a description will be made on an outline of a diagnostic support system according to a first embodiment of the present invention. In the diagnostic support system of the present invention, a learned model is used. The learned model is generated to be able to output feature value information of an input image from such an image by performing the machine learning of comparison images of a case or the like, which are acquired in advance. The feature value information of an examination image to be examined is calculated by using this learned model, and similarity between the examination image and the comparison image is displayed on the basis of this feature value information. Various methods are available as a method for presenting the similarity between the examination image and the comparison image. For example, in one of the methods, information of a multidimensional parameter serves as the feature value information, and a distance on a feature value space with this multidimensional parameter as a coordinate is identified and displayed as a value that represents similarity between the examination image and the comparison image. In another method, the multidimensional parameter or linguistic expression information serves as the feature value information, the plural comparison images and the examination image are arranged in the virtual space that is created by using this feature value information to create a drawing, and such a drawing is displayed as a virtual space image. In this case, it is preferable that the respective comparison image and a lesion feature information (label information) pertaining to the respective comparison image are additionally displayed when any of the comparison images or a region on the virtual space image is selected. Here, the virtual space is an imaginary space (for displaying) including the number of displayable dimensions for visual recognition of the similarity between the examination image and the comparison image using the feature value information. The virtual space image is an image in which the similarity between the examination image and the comparison image is displayed in a visually recognizable manner by plotting positions in the feature value space thereof on this virtual space. The lesion feature information (the label information) includes, but not limited to, a position of a tumor (a position thereof in a breast, a position thereof in a mammary gland, or a distance to a skin), whether solid or cystic, presence or absence of a structure in the tumor, presence or absence of a posterior shadow, an aspect ratio (a ratio among lengths of an a-axis, a b-axis, and a c-axis in a case of being approximated as a spheroid), a property of the boundary (whether an echo in a boundary part is a hyper-echo or a low echo, or whether a shape of the boundary is smooth or not smooth), presence or absence of architectural distortion of surrounding normal tissues, presence or absence of plural tumor masses such as a daughter nodule, and presence or absence of calcification. Meanwhile, in the machine learning, the feature value space is consequently determined such that the image having plural types of the label information is separated the most. Accordingly, the feature is not always the same as the conventional feature that has been used for the determination by a clinician.

When the similarity (particularly, a distance between the comparison image and the examination image in the feature value space or the virtual space) displayed by this diagnostic support system can be checked, an existing diagnostic criteria recognizable by the person and the feature value information calculated by using the machine learning are combined. Thus, this diagnostic support system can support a user who is short on experience in interpretation of the images to improve efficiency and accuracy of a diagnosis. In addition, in the case where the diagnostic support system also makes determinations on presence or absence of the lesion and benignancy/malignancy thereof, the information on the similarity is used as information serving as basis. In this way, it is possible to check adequacy of a determination result by the diagnostic support system. The determination result described herein means display of the comparison image as the similar image, a probability of cancer (a malignant tumor) in the examination image based on these types of the information, and the like.

A detailed description will be made on the first embodiment as a preferred aspect of the present invention. FIG. 1 is a schematic block diagram illustrating a configuration of a diagnostic support system 1S according to the first embodiment of the present invention. The diagnostic support system 1S includes, at least, a diagnostic support device 2 and a pre-acquired data storage unit 3. The diagnostic support device 2 is connected to an imaging device 4. Connection between the diagnostic support device 2 and the imaging device 4 may be wired connection or wireless connection, and the connection to the remote imaging device 4 may be connection via the Internet. In addition, the imaging device 4 and the diagnostic support device 2 may be integrally incorporated as the single device.

The imaging device 4 captures a medical image of a subject to acquire an internal information image. For example, an ultrasonic diagnostic device (as disclosed in WO 2017/051903) is applied to the imaging device 4 exemplified in this embodiment.

This imaging device 4 is primarily used for examination of a tumor of breast cancer and can acquire internal information of the breast as the subject as a 3D image. The breast as the subject is inserted in a subject insertion section of a probe 31. An ultrasonic array arranged around the subject vertically scans the subject while transmitting/receiving ultrasound, so as to generate an examination image of the internal information of the subject. In addition to the probe 31, the imaging device 4 includes units such as a transceiving control unit 32, an image generation unit 33, a device control unit 34, and a storage unit 35.

The transceiving control unit 32 controls transmission and reception of an ultrasound signal from the probe 31. The device control unit 34 controls operation of the imaging device 4, including scanning of the probe 31, and the like. The image generation unit 33 reconstructs the ultrasound signal received by the transceiving control unit 32 and generates the examination image. Here, the generated examination image may be an image showing the entire breast as the subject or may be an image showing part of the subject, for example, only a lesion estimated area.

The storage unit 35 stores and accumulates the acquired received signal, subject information, captured image, and the like in a manner capable of calling up as needed. The storage unit 35 is a known storage device such as a HDD or a SSD, or the like. The storage unit 35 can be incorporated in the imaging device 4 as shown in the drawing or can be substituted by an external server (not shown) of the imaging device 4 or the like when being connected to this external server.

In this embodiment, a description will hereinafter be made on ultrasonic image diagnostic support for breast cancer in a three-dimensional medical image of the breast that is acquired by the imaging device 4. It is needless to say that a target of the present invention is not limited to a diagnosis of breast cancer by the device. For example, the internal information of a target area may be that of a head, a body, a limb, or the like. In addition, the diagnosis thereof is not limited to an ultrasound diagnosis. The ultrasound diagnosis is also combined with two-dimensional or three-dimensional CT or MRI or another imaging technology.

As described above, the diagnostic support system 1S in the embodiment includes the diagnostic support device 2 and the pre-acquired data storage unit 3. The diagnostic support device 2 includes, at least, a communication control unit 11, a control unit 12, a calculation unit 13, a virtual space data storage unit 14, a display control unit 15, a display unit 16, and an input unit 17, and performs the image diagnostic support by using the examination image acquired by the imaging device 4.

The pre-acquired data storage unit 3 stores a group of plural comparison images as comparison targets at the time of diagnosing the examination image. Each of the images is an internal biological information image that is acquired in advance, and can be two- or three-dimensional image data or image data that includes a case image composed of (radiofrequency or high-frequency) data before being converted into the image data. The plural comparison images that are stored herein further include feature value information with which a degree of similarity between the plural comparison images can be identified. However, the comparison images in the pre-acquired data storage unit 3 do not necessarily and directly have this feature value information (in detail, N-dimensional parameter information, which will be described below). This feature value information only needs to be derived from data on the comparison images by using the calculation unit 13, which will be also described below, for example. Furthermore, as this comparison image, in addition to the case image, a focal case simulation image that is acquired by computer calculation, intermediate data on the focal case simulation image, an image of a finding or a diagnostic criteria, an image of a normal tissue, or the like can be adopted. Moreover, the comparison image may be a captured image of the entire breast as the subject or a captured image of the part of the subject, for example, only the lesion estimated area. In this embodiment, it is assumed that ultrasound images are compared. However, the comparison image is not limited to such an ultrasound image, and a medical image that is acquired by another type such as X-ray CT may be used as the comparison image.

In addition, the pre-acquired data storage unit 3 stores information indicating biological tissue information and shape information (of a biological tissue) of each of the comparison images, in detail, label information including the lesion feature information of each of the comparison images, and these are linked to each of the comparison images. The label information including the lesion feature information is used for diagnostic support of the subject, and is also read to indicate an attribute of the comparison image at the time of displaying the comparison image as an image showing basis for a diagnostic support determination result. The label information including the lesion feature information includes diagnostic information by a doctor, biological information of the subject, and the like such as the finding or a diagnosis result that is determined comprehensively on the basis of the diagnostic criteria or the plural diagnostic criterion, a pathological diagnosis by needle biopsy or the like, a temporal change in the subject, and history of treatment. This label information has to be linked to each of the plural comparison images in the pre-acquired data storage unit 3. However, the label information does not always have to be linked to all the comparison images in the pre-acquired data storage unit 3.

In addition, since the label information including the image lesion feature information is linked to each of these comparison images, the comparison images constitute, as tagged supervised data, a learning data set of a learned model for the calculation unit 13, which will be described below.

In this embodiment, the pre-acquired data storage unit 3 is arranged in the server that is connected to the outside of the diagnostic support device 2, or the like. However, the pre-acquired data storage unit 3 may be incorporated in the diagnostic support device 2 (not shown). In addition, the plural comparison images in the pre-acquired data storage unit 3 may be provided to the pre-acquired data storage unit 3 via a network or a portable recording medium.

The diagnostic support device 2 is configured to include a CPU, a GPU, main memory, another LSI, ROM, RAM, and the like of the control unit 12. Operation thereof is performed with a diagnostic support program that is loaded to the main memory, or the like. That is, the diagnostic support device 2 can be realized by using any of various computers (calculation resources) such as a personal computer (PC), a main frame, a work station, and a cloud computing system.

In the case where each function unit of the diagnostic support device 2 is realized by software, the diagnostic support device 2 is realized by executing a command of a program as software for implementing each function. As a recording medium storing this program, a "non-temporal tangible medium" such as a CD, a DVD, semiconductor memory, or a programmable logic circuit can be used. In addition, this program can be supplied to the computer in the diagnostic support device 2 via a specified transmission medium (a communication network, a broadcast wave, or the like) capable of transmitting the program.

The communication control unit 11 is an interface for controlling the transmission and the reception of the data between the image diagnostic device 4 and the pre-acquired data storage unit 3. The communication control unit 11 primarily acquires the examination image, the group of comparison images, the label information including the lesion feature information of the comparison images, and the like.

The control unit 12 includes at least processors such as the CPU and the GPU, and controls all the function units in the diagnostic support device 2. In particular, in this embodiment, this control unit 12 has a function of identifying the similarity between the examination image and each of the plural comparison images. Such a function will be described below.

The calculation unit 13 calculates and acquires the feature value information of the examination image, which is received via the communication control unit 11, and of the plural comparison images as needed. This calculation unit 13 constitutes a so-called classifier and has the specified learned model therein. This learned model is generated by a well-known machine learning method, for example, through supervised learning using a neural network (preferably including a convolutional neural network (CNN)) model. In addition, this learned model is a learned model that has learned (trained) to output the feature value information to a neuron in an output layer by inputting the data on the examination image and the plural comparison images into a neuron in an input layer thereof. The machine learning technique for the learned model is not limited to the above. Any of techniques such as a support vector machine (SVM), a model tree, a decision tree, multiple linear regression, locally weighted regression, and an established search method can be used alternatively, or the methods can appropriately be combined and used.

This learned model for the calculation unit 13 is acquired by learning some or all of the plural comparison images, which are stored in the pre-acquired data storage unit 3 and include the mutually-linked label information, as the learning data set. Accordingly, it should particularly be noted that the data on this learned model, which is input to the neuron in the input layer, has to be in the same format as the data on the plural comparison images. For example, in the case where the comparison image includes the three-dimensional image data, the data input to each of the neurons in the input layer can be a (eight-bit) gray scale value of each voxel that constitutes this three-dimensional image data, for example. Similarly, in the case where the comparison image includes the two-dimensional image data, the data input to each of the neurons in the input layer can be the gray scale value of each pixel that constitutes this two-dimensional image data, for example. The data input to each of the neurons in the input layer is not limited thereto, and can appropriately be changed according to the format of the data constituting the comparison image, presence or absence of additional information, or the like.

In this calculation unit 13, the feature value information in the learned model which is output to the output layer includes information with which a feature of the image can be identified in a machine learning network, but the format and the number of the information are not limited. In this embodiment, the feature value information is information including the multidimensional, for example, N (a natural number equal to or larger than 2) dimensional parameter that is the feature value identified at the learning stage. As described above, this learned model is generated through the machine learning of the learning data set that includes the comparison images and the label information linked thereto. The label information can be classified by a value such as the diagnostic criteria that can be recognized by the user, for example, a value indicating presence or absence of a whitened area in specified size or larger in the image, the size and a location of the whitened area, a thickness of a peripheral vein, or the like.

The virtual space data storage unit 14 generates a specified virtual space (a first virtual space) by using the N-dimensional parameter that is output from the learned model provided in the calculation unit 17. This virtual space data storage unit 14 stores various types of data used to plot the plural comparison images and the examination image at specified coordinate positions on this virtual space. The various types of the data described herein include a calculation formula and the like used to plot the image on the displayable virtual space in the dimensional number (for example, first to third dimensions) by adjusting the value of the N-dimensional parameter calculated by the calculation unit 17. This calculation formula is specifically exemplified. For example, in the case where the calculation unit 17 desires to output 10-dimensional parameter information as the feature value information of the plural comparison images and to plot the plural comparison images on the two-dimensional space as the virtual space, a calculation formula that specifies a 2-dimensional value by dividing the 10-dimensional parameter information into two, multiplying the divided 10-dimensional parameter information by a preset weight value when needed, and adding the divided 10-dimensional parameter information to each other, a calculation formula that specifies the 2-dimensional value by using a well-known multivariate analysis technique for the 10-dimensional parameter information, or the like may be adopted. Needless to say, in the case where the plural comparison images are plotted on such a virtual space, the coordinate of each of the comparison images needs to have a specified correlation with relevant information (that is, the diagnosis result) that is linked to respective one of the comparison images. Accordingly, for example, the parameter information, which is output from the calculation unit 17, the weight value included in the calculation formula stored in this virtual space data storage unit 14, and the like are adjusted on the basis of the diagnosis result of the comparison image. Needless to say, in the case where the dimensional number of the N-dimensional parameter output from the calculation unit 17 is small (for example, the three dimensions), the comparison images and the examination image can be plotted on the virtual space (in the same dimensional number) without performing an additional calculation and the like. As a result, in this case, the virtual space and the feature value space constitute the same space.

In addition, in this embodiment, it is exemplified that the calculation unit 13 calculates the N-dimensional parameter of each of the plural comparison images from moment to moment. However, since the comparison images are not images that are frequently added, changed, or the like, the plural comparison images and the N-dimensional parameters thereof may be stored in this virtual space data storage unit 14. In this way, it is possible to reduce a calculation amount by the calculation unit 13 at the time of generating the virtual space and thus to reduce burdens on the calculation unit 13 and the control unit 12.

Here, a brief description will be made on an example of a similarity identification technique by the control unit 12. The control unit 12 calls the N-dimensional parameters as the feature value information of the plural comparison images calculated by the calculation unit 13 and the N-dimensional parameter as the feature value information of the examination image also calculated by the calculation unit 13, and plots the N-dimensional parameters on the feature value space configured as the N-dimensional space with the N-dimensional parameters being the coordinates. Then, the labeled diagnostic criterion and the label information of the diagnosis result are read into the comparison images arranged on this feature value space. In this way, for example, a boundary line between benignancy/malignancy is drawn on the feature value space. Here, the feature value space has the N number of axes. Accordingly, in the case where the coordinate of the examination image on the feature value space is a vector $X = (x_1, x_2, ..., x_N)$, and the coordinate of the comparison image, in which a distance is a calculation target, on the feature value space is a vector $Y = (y_1, y_2, ..., y_N)$, a distance L is calculated by a mathematical formula (ii) expressed as follows. Furthermore, a mathematical formula (iii) may be used by weighing each component on the feature value space.

$$L = \sqrt{\sum_{i=1}^{N}(x_i - y_i)^2} \qquad \cdots \text{(ii)}$$

$$L = \sqrt{\sum_{i=1}^{N} w_i (x_i - y_i)^2} \qquad \cdots \text{(iii)}$$

The distance L that is identified herein is a value representing the similarity between the examination image and each of the plural comparison images. In addition, a distance between the sets such as a benign tumor (a tumor mass) and a malignant tumor is calculated by a sum of the distances between the comparison images belonging to the set. Needless to say, the set and the distance may not be calculated as the sum of the distances of all the comparison images that belong to the set. Instead, the top M number of the comparison images in the set may be picked from the comparison images located near the examination image, and a sum of the distances of the picked comparison images may be calculated. Alternatively, the distances to a boundary line may be calculated. Thus, the calculations of the set and the distance are not limited. The distance L is the value that is identified as the distance on the feature value space. However, the space in which the distance is measured is not limited to this feature value space. For example, each of the comparison images and the examination image may be plotted on an m-dimensional ($1 \leq m < N$) virtual space, which is acquired with reference to the virtual space data storage unit 14, by using the calculated N-dimensional parameter. Then, the distance on this virtual space may be calculated by using a similar mathematical formula to the above-described mathematical formula (ii) or mathematical formula (iii). The adoption of the distance on the virtual space as the value indicative of the similarity between the examination image and each of the comparison images is especially advantageous for a case where a virtual space image 45, which will be described below, is displayed. In detail, in this case, the distance between the examination image and each of the comparison images displayed in the virtual space image matches the distance as the value indicative of the similarity between the examination image and each of the plural comparison images. Thus, the similarity therebetween can accurately be comprehended simply by looking at the virtual space image 45.

The accuracy of the feature value space or the virtual space depends on: the comparison image as the learning data set used to generate the learned model for the calculation unit 13; and quality and an amount of the label information that is linked to this comparison image. In the case where the examination image is arranged at a position, a distance of which from a set of the benignant comparison images is equal to a distance of which from a set of the malignant comparison images, on the feature value space or the virtual space, an attribute distribution map including benignancy, malignancy, and the like is changed by increasing the number of the comparison images. As a result, the examination image that is located on a boundary with the different attribute can further easily be determined. Meanwhile, since the types and the number of the comparison images are increased, there is possible appearance of a portion, in which the sets of the different attributes overlap each other, on the feature value space or the virtual space. In the case where the determination on the feature value space or the virtual space is difficult, just as described, a degree of vagueness of the diagnostic determination is increased, which loses meaning of the numerical value of the probability of the determination and the like. In such a case, the diagnostic determination is not made by the machine, only the virtual space image is displayed, and the determination by the doctor himself/herself is added. In this way, it is possible to make the further accurate diagnostic determination. As it is understood from the above description, the number of the comparison images affects the output of the calculation unit. Accordingly, in order to prevent a change in the output of the diagnostic support device 2 according to use thereof, when this learned model is generated, only batch learning that uses the learning data set prepared in advance is adopted, and so-called online learning, in which the learned model is updated by using the examination image and the like for learning, does not have to be adopted.

The display control unit 15 generates the image used as the basis for the diagnostic determination result by the control unit 12. The image used as the basis may only be the numerical value that represents the similarity identified by the control unit 12, may adopt a display layout based on the similarity, or may be the virtual space image that is generated when the control unit 12 arranges the plural comparison images and the examination image on the feature value space or the virtual space by taking the virtual space data storage unit 14 into consideration, for example. In addition to the above, the image used as the basis may be a display example that displays a correlation of an Euclidean distance between the examination image and each of the comparison images on the feature value space or the virtual space, or may be a display example that displays a function of the coordinate of each of the comparison images. However, the image used as the basis is not limited thereto. In addition, this display control unit 15 can add a specified visual effect to a point indicative of the comparison image that is plotted on the virtual space. As this visual effect, for example, the label information, such as the lesion feature information, that is linked to each comparison image is taken into consideration. Then, a point of the comparison image (a malignant case image), the label information of which includes information including the "malignant tumor," can be shown in red, a point of the comparison image (a benign case image), the label information of which includes information including the "benign tumor," can be shown in blue, and a point of the comparison image (a normal tissue image), the label information of which includes information of being "normal," can be shown in black. The visual effect is not limited to any of the above-described effects, and any of various other visual effects can be adopted. In this way, when the similarity between the examination image and the comparison image is displayed in such a format that allows the doctor or the like to comprehend the similarity through visual sensation, the relative similarity of each of the plural comparison images to the examination image is clarified. With this as the basis for the diagnostic determination by the machine, the user determines reliability of the diagnostic determination by the diagnostic support system 1S, and can thereby effectively derive the diagnosis result of the image interpretation.

The display unit 16 is a display device such as a display. This display unit 16 displays the examination image acquired by the communication control unit 11, the determination result acquired by the control unit 12, and the information on the similarity (for example, the virtual space image) that is generated by the display control unit 15 and serves as the basis for the determination result. That is, the display unit 16 not only displays the determination result acquired from the comparison (for example, the specified number of the similar comparison images or a speculation result by a speculation unit 18, which will be described below) but also displays the value of the distance on the feature value space, which serves as the basis for the derivation of the determination result, the virtual space image, and the like. The display unit 16 also synthesizes the images or the like in order to display required information for the operation of the diagnostic support system 1S, and the like. A detailed description on the display will be made below with reference to schematic views. In this embodiment, the display unit 16 is incorporated into the diagnostic support device 2. However, the display unit 16 may be connected to a display unit of an external PC terminal, a mobile terminal, or the like via the Internet.

The input unit 17 is a keyboard, a touchscreen, a mouse, or the like for the operation. The input unit 17 can be used to make input for the operation of the diagnostic support device 2, designate an examination area in the examination image, select a display pattern, enter a finding comment to a workstation, and the like.

The diagnostic support device 2 according to this embodiment may further include the speculation unit 18 and a pre-processing unit 19.

The speculation unit 18 speculates whether specified preset biological information, in detail, the malignant tumor or the like is included in the examination image. Similar to the calculation unit 13, this speculation unit 18 includes a learned model. Similar to the learned model for the calculation unit 13, this learned model in the speculation unit 18 is generated by the well-known machine learning method, for example, through the supervised learning using the neural network model. In addition, this learned model can be generated by performing the machine learning using the learning data set, in which the plural comparison images stored in the pre-acquired data storage unit 2 and presence or absence of the label information linked to each of the comparison images, in particular, the biological information as the diagnosis result are provided as the set, for example. The thus-generated learned model is a learned model that has learned to output whether the specified biological information is included in the neuron of the output layer or a probability that the specified biological information is included (also referred to as a confidence value) by inputting (the image data of) the examination image to the neuron of the input layer. Instead of the image data as the image data of the examination image, the data that is input to the neuron in the input layer may be the N-dimensional parameter information of the examination image calculated by the calculation unit 13 or RF data of the examination image. The machine learning method for this learned model is not limited to the above. Any of methods such as the SVM, the model tree, the decision tree, the multiple linear regression, the locally weighted regression, and the established search method can be used alternatively, or the methods can appropriately be combined and used. In addition, the biological information described herein is not limited to the malignant tumor but also includes the benign tumor and an artifact. The learned model in the speculation unit 18 may include any of these types of the biological information or may output the probability that any of these types of the biological information is included in the examination image. By adopting such a speculation unit 18, the diagnostic support device 2 can provide the user with the probability that the specified biological information is included in the examination image as the determination result of the diagnostic support system 1S, in addition to the similarity between the examination image and each of the plural comparison images. As a result, it is possible to further improve diagnostic efficiency of the doctor.

The pre-processing unit 19 adjusts the data format of the data on the examination image, which is received by the communication control unit 11, before the calculation by the calculation unit 13 so that the feature value information thereof can be calculated by the calculation unit 13. This pre-processing includes various types of processing in addition to processing (for example, noise filtering, data volume adjustment, FFT, or the like) that is normally executed in the technical field of a machine learning device. Specific examples of the various types of this processing are: processing to generate one or more pieces of two-dimensional slice data (automatically or via the operation by the user) from the three-dimensional image data in the case where the learned model in the calculation unit 13 has learned to output the feature value information by inputting the two-dimensional image data and where the examination image received by the communication control unit 11 is the three-dimensional image data; processing to generate the two-dimensional image data from RF data in the case where the learned model in the calculation unit 13 has learned to output the feature value information by inputting the two-dimensional image data and where the examination image received by the communication control unit 11 is the RF data; and processing to generate the linguistic expression information from the image data in the case where the learned model in the calculation unit 13 has learned to output the feature value information by inputting the linguistic expression information, which will be described below, and where the examination image received by the communication control unit 11 is the two-dimensional or three-dimensional image data. The input/output information of the learned model is specified according to the data configuration of the learning data set at the time of the machine learning. Accordingly, by adopting such a pre-processing unit 19, the plural learned models no longer have to be prepared according to the data format of the examination image.

FIG. 2 is a flowchart showing an operation outline of the diagnostic support system according to the first embodiment of the present invention. The diagnostic support method described herein is implemented by any of various computers constituting the diagnostic support system, and includes a comparison image storage step (S1000), a comparison image multidimensional parameter generation step (S1100), an examination image acquisition step (S1200), a comparison image and multidimensional parameter acquisition step (S1300), an examination image multidimensional parameter calculation step (S1400), a virtual space image formation display step (S1500), and a comparison image display step (S1600). The diagnostic support method also includes steps, which are not shown, such as recording step, storing step, accumulating step, and calculating step.

First, as advance preparation for the diagnostic support, in the comparison image storage step (S1000), the plural comparison images collected in advance are stored in the pre-acquired data storage unit 3. Each of the plural comparison images collected herein is primarily composed of the three-dimensional image data, for example. Next, in the comparison image multidimensional parameter generation step (S11100), the multidimensional (N-dimensional) parameter as the feature value information of each of the comparison images is generated by the calculation unit 13 and stored in the virtual space data storage unit 14. Here, it can also be configured to generate the multidimensional parameters of the plural comparison images in advance and to store each of the comparison images and this multidimensional parameter thereof as a set in the pre-acquired data storage unit 3. In such a case, this comparison image multidimensional parameter generation step (S1100) can be omitted. In addition, the above-described comparison image storage step (S1000) and the comparison image multidimensional parameter generation step (S110) may be executed for each examination. However, since a content of the comparison image is not frequently changed, the above-described comparison image storage step (S1000) and the comparison image multidimensional parameter generation step (S1100) may be executed only at update timing of the content of the comparison image.

Next, in the examination image acquisition step (S1200), the examination image that is captured by the imaging device 4 and serves as an examination target is acquired via the communication control unit 11. This examination data is also composed of the three-dimensional image data, for example. Furthermore, in the comparison image and multidimensional parameter acquisition step (S1300), the pre-acquired data storage unit 3 or the virtual space data storage unit 14 acquires the set of the plural comparison images and the multidimensional parameters corresponding thereto. Then, the calculation unit 13 calculates the multidimensional parameter as the feature value information of the acquired examination image (the examination image multidimensional parameter calculation step (S1400)). Furthermore, in the virtual space image formation display step (S1500), the control unit 12 and the display control unit 15 generate a virtual space image to be displayed on the basis of various types of the data in the virtual space data storage unit 14, and the display unit 16 displays the virtual space image. In this embodiment, the comparison image and the examination image are plotted at particular coordinate positions on the virtual space image, just as described. In this way, the similarity therebetween is displayed. Furthermore, by selecting the coordinate of the comparison image on the virtual space image, the user can preferably display the comparison image and the label information such as the lesion feature information (the comparison image display step (S1600)).

As it has been described so far, in the diagnostic support system according to this embodiment, when the similar image, which is determined to be similar to the examination image, of the comparison images is displayed as the determination result, the information on the similarity between the images, such as the virtual space images, is displayed as the basis for the selection. In this way, it is possible for the user to check adequacy of the determination by this system, resulting in an improvement of diagnosis efficiency.

The examination image acquisition step (S1200) and the comparison image including the multidimensional parameter acquisition step (S1300) may be executed in parallel or may be executed sequentially. In addition, instead of the virtual space image formation display step (S1500), the distance on the feature value space can be identified from the multidimensional parameter of each of the examination image and the plural comparison images, and the display unit 16 can display such a distance as a value representing the similarity.

Furthermore, in the case where the diagnostic support device is used for an educational purpose, or the like, the user can select a mode, in which the diagnostic determination by the machine is not displayed, in advance.

The virtual space data storage unit 14 that uses the N-dimensional parameter as the feature value information to generate the first virtual space has been exemplified. However, the virtual space data storage unit 14 according to this embodiment is not limited thereto. As another aspect, the virtual space data storage unit 14 may adopt the linguistic expression information that corresponds, as the feature value information, to each of the images and may generate a linguistic space including this linguistic expression information as a second virtual space. In this case, the display unit 16 displays the similarity that is calculated with the distance of each of the examination image and the plural comparison images on the linguistic space being a reference.

For example, the linguistic space can be based on linguistic expression such as image interpretation report expression or the like that is linked to the comparison image. First, the control unit 12 uses the learned model for the calculation unit 13 to convert the comparison image into an image interpretation language included in the comparison image, and reads the image interpretation language as language information. Similarly, the control unit 12 also converts the examination image into the image interpretation language and reads the image interpretation language. For example, the image interpretation language is the language information or the finding that is determined from an image indicating that progress of the tumor is "2" or the like, or is language data that is converted into natural language data or the like. Here, the finding may include similar information to the above-described lesion feature information, that is, may include the information on the position of the tumor (the position thereof in the breast, the position thereof in the mammary gland, the distance to the skin), whether solid or cystic, presence or absence of the structure in the tumor, the presence or the absence of the posterior shadow, the aspect ratio (the ratio among the lengths of the a-axis, the b-axis, and the c-axis in the case of being approximated as the spheroid), the property of the boundary (whether the echo in the boundary part is the hyper-echo or the low echo, or whether the shape of the boundary is smooth or not smooth), the presence or the absence of the architectural distortion of surrounding the normal tissues, the presence or the absence of the plural tumor masses such as the daughter nodule, and the presence or the absence of the calcification. Thereafter, the control unit 12 replaces the examination image and the comparison image with indexes on the linguistic space (the second virtual space), and the indexes are displayed on the linguistic space so as to visually recognize the similarity between the examination image and the comparison image. In addition, the similarity between the examination image and the comparison image can be identified as a numerical value by measuring distance distribution of the examination image and the comparison image.

In this example, the learned model is provided. The learned model can verbalize and extract the feature value that corresponds to the existing diagnostic criteria for the lesion estimated area in the examination image by performing the machine learning using the learning data set that includes the comparison images and the label information including the lesion feature information. As a result, it is possible to verbalize new diagnostic criteria that are not obvious for human eyes.

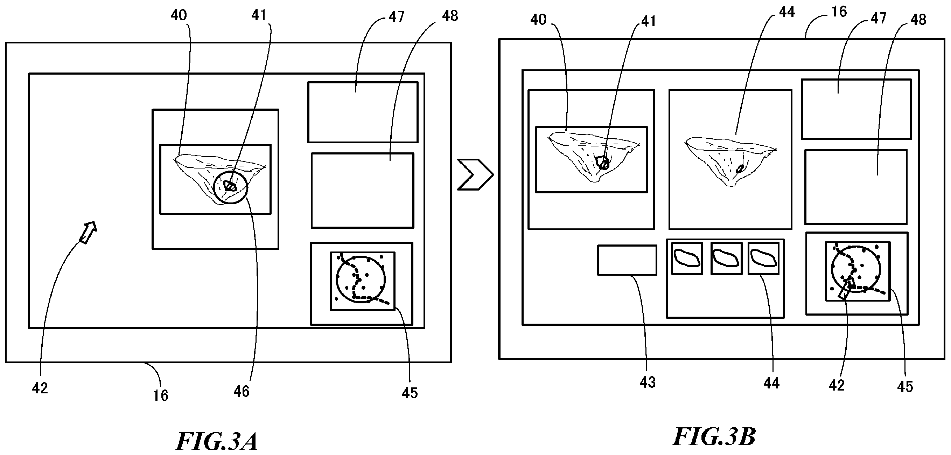

Next, a specific description will be made on a display content of the display unit 16 in this embodiment. FIG. 3 includes schematic views illustrating an example of the display in the display unit 16. FIG. 3A illustrates a three-dimensional examination image 40 of the breast as the subject, which is acquired from the imaging device 4 through the communication control unit 11, in a center portion of the display unit 16. In this examination image 40, there exists a feature area 41 with a high possibility as the tumor that should carefully be observed. At this time, for a purpose of enhancing visibility, such a visual effect may be added that an area including the feature area 41 is circled as a lesion estimated area 46. In addition, as a method for identifying this feature area 41 and the lesion estimated area 46, the feature area 41 and the lesion estimated area 46 are not only selected by the user but can also be identified automatically by using well-known image recognition technology or the machine learning technique. Furthermore, as the machine learning technique used herein, for example, the neural network model, in detail, such a learned model can be adopted that the image data is input to the neuron in the input layer so as to output, to the output layer, a coordinate that indicates an area matching the preset biological information. In the display of the display unit 16 shown in FIG. 3A, the virtual space image 45 that indicates the similarity between the comparison image and the examination image and is composed of the two-dimensional map is displayed.

FIG. 3B is a schematic view showing an example of the display after the pointer 42 designates the coordinate of the particular comparison image in the virtual space image 45. When the coordinate of the comparison image is designated, this designated comparison image 44 is displayed, as the similar case, in the center portion of the display unit 16. Alternatively, another comparison image 44 that is arranged from the examination image by a specified distance on the virtual space may be juxtaposed and displayed with the designated comparison image 44. The display unit 16 also displays the determination result 43 of the feature area 41. This determination result 43 includes, but not limited to, a probability that the biological information speculated by the speculation unit 18, a benignancy/malignancy differential determination, and the corresponding diagnosis name. In addition, as the display method of the determination result 43, a method for using a specified visual effect such as highlighting the feature area 41a instead of text information may be adopted.

As described above, the virtual space image 45 is displayed as the image showing the basis for the determination result. Thus, the doctor or the like can determine the vagueness of the determination result and can use the virtual space image 45 to confirm the diagnosis. In addition, since some of the comparison images corresponding to the similar case are also displayed with the determination result 43 in the display unit 16, it is possible to improve the diagnostic efficiency.

In addition to the various images, for example, a patient information display section 47, an imaging condition display section 48, and the like are also displayed on the display of the display unit 16. The types, the arrangement, and the like of the display information are only illustrative and thus are not limited thereto. In the case where the examination image and the comparison image are compared in the format of the three-dimensional image, the displayed comparison image may be three-dimensional.

In addition, a window for displaying the various types of the data does not have to be one. The examination image 40, the virtual space image 45, and the like may appear on another window or tab display according to an input command.

Next, referring to FIG. 4 to FIG. 7, a description will be made on another display example of the image showing the basis. The image showing the basis for the determination result by the diagnostic support system 1S is displayed as the virtual space image 45 in FIG. 3, for example. Alternatively, the plural virtual space images may be juxtaposed.

FIG. 4 includes schematic views illustrating an example of a case where map display is adopted as the virtual space image 45 that is generated by projecting the feature value space and the feature value space. FIG. 4A illustrates a feature value space 50. In order to simplify the description, this feature value space 50 illustrated in FIG. 4A exemplifies a case where the feature value information output by the calculation unit 13 is composed of the two-dimensional parameters. Then, these two parameters constitute the two-dimensional space by setting a vertical axis and a horizontal axis. A point 53 indicating the examination image is arranged on the feature value space 50 on the basis of the two-dimensional parameter acquired as a result of the calculation by the calculation unit 13. The number of the dimensions of the feature value space 50 described herein is not limited. Similar to the point 53 indicating the examination image, points corresponding to the plural comparison images are plotted on the feature value space 50 in FIG. 4A. As an example, points 51 corresponding to the plural malignant case images are indicated by black circles, and points 52 corresponding to the plural benign case images are indicated by white circles. Here, the malignant case means the malignant tumor such as breast cancer, and the malignant case image is the comparison image including the malignant tumor. Similarly, the benign case means the benign tumor, and the benign case image is the comparison image including the benign tumor. In addition, in this feature value space 50, a benignancy/malignancy discriminant line 54 can be provided on the basis of arrangement of these points 51, 52. In this way, for example, as the above-described distance indicating the similarity between the examination image and the comparison image, it is possible to identify a distance between a position of the point 53 and the benignancy/malignancy discriminant line 54 in the feature value space 50 as well as the number and the feature (whether the benign case or the malignant case) of the comparison image that exists within a specified range 55 of the distance from the point 53 corresponding to the examination image. By displaying these types of the information, the diagnostic support for the examination image can be provided to the doctor. The display control unit 15 can generate the one-dimensional, two-dimensional, or three-dimensional virtual space image as the image showing the basis for the determination result 43 on the basis of this feature value space 50, and can display the virtual space image on the display unit 16.

FIG. 4B illustrates an example of a one-dimensional virtual space image 56 generated on the basis of the information such as the feature value space 50 illustrated in FIG. 4A. The comparison image is added with the visual effect and plotted such that it can be understood whether such a comparison image shows the malignant case or shows the benign case. In this one-dimensional virtual space image 56, the point 53 corresponding to the examination image is arranged at a center, and the points 51, 52 corresponding to the comparison images are displayed in an area, a right-left direction of which is set as a benign direction (a + direction) and a malignant direction (a - direction). The comparison image may be displayed when any of the points 51, 52 corresponding to the comparison images is selected by a pointer, which is not shown, or the like. Display positions are based on the Euclidean distance from the point 53 corresponding to the examination image, for example. The display in FIG. 4B is advantageous in a point that the relative Euclidean distance is easily discriminated.

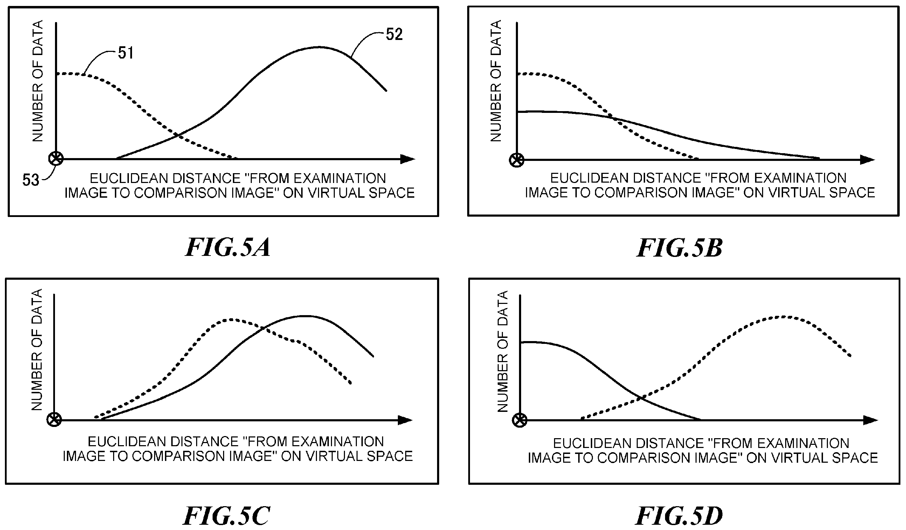

FIG. 5 includes schematic graphs illustrating an example of a case where a histogram display is adopted as the virtual space image 45. FIG. 5A to FIG. 5B each illustrates histograms of the number and the distance of the comparison image that exists within a specified range 55 centered on the point 53 corresponding to the examination image in the feature value space 50. Here, in each of FIG. 5A to FIG. 5D, a horizontal axis represents the Euclidean distance from the point 53, which corresponds to the examination image, to the point corresponding to the comparison image in the feature value space 50, and a vertical axis represents the number (the number of the data of) the comparison image. In addition, a dotted line represents a line indicating transition in the number of the point 61 corresponding to the malignant case image, and a solid line represents a line indicating transition in the number of the point 62 corresponding to the benign case image. Furthermore, the point 53 corresponding to the examination image that serves as a benchmark is set at a position of an origin of the histogram.

It can be understood that, in the histogram illustrated in FIG. 5A, the large number of the points 51 corresponding to the malignant case image are present on a near side of the point 53 corresponding to the examination image and the large number of the points 52 corresponding to the benign case image are present on a far side. Accordingly, it is understood from this image that there is a high possibility that the examination image shows the malignant case. On the contrary, it is understood that, in the histogram illustrated in FIG. 5D, the probability of the point 53 corresponding to the examination image is the benign case is high. In addition, it is suggested that, in the histogram illustrated in FIG. 5B, the point 53 corresponding to the examination image is far from both of the point 51 corresponding to the malignant case image and the point 52 corresponding to the benign case image and thus there is a possibility that the tumor or the like does not exist in the examination image. However, in the histogram illustrated in FIG. 5C, the large number of the comparison images of both of the point 52 corresponding to the benign case image and the point 51 corresponding to the malignant case image exist at positions near the point 53 corresponding to the examination image. Accordingly, in this case, it is suggested that the accuracy of the support information by the diagnostic support system 1S is low and further another examination means such as the pathological diagnosis is necessary. This example display has such an advantage that the doctor can comprehend, at a glance, overall relationship between the examination image and the large number of the comparison images. In addition to the method for displaying all the comparison images, there is a method for only displaying the comparison image from a different perspective from the image information, and such a comparison image has a similar property to the examination target, such as whether dense breast or not, age, or genetic information.

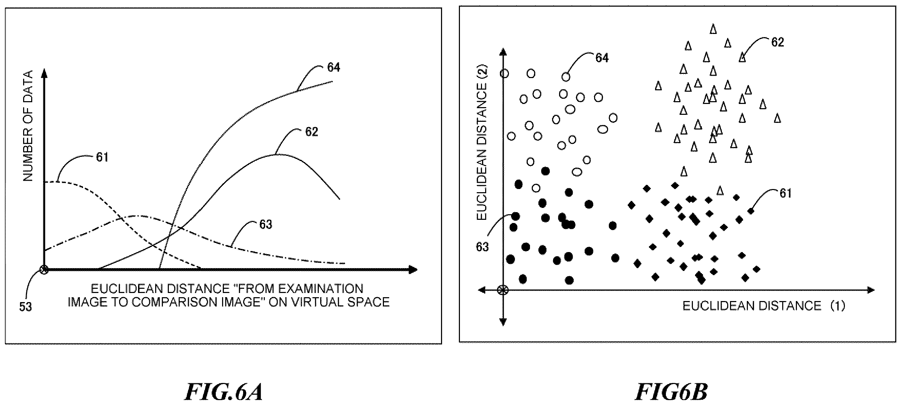

FIG. 6 includes schematic graphs illustrating another example of the case where the histogram display is adopted as the virtual space image 45. FIG. 6A illustrates histograms of correlations of the data number and the distance between the comparison image in each attribute and the examination image. Here, four types of the comparison images are provided. These four types are: a point 61 corresponding to the malignant case image; a point 62 corresponding to the benign case image; a point 63 corresponding to an artifact image; and a point 64 corresponding to the normal tissue. These types are identified on the basis of the label information linked to the comparison images. Here, the artifact means a virtual image that is neither the tumor nor the tumor mass and is captured due to noise or the like. The artifact image is the comparison image including this artifact. The normal tissue image is the comparison image including none of the tumor, the tumor mass, and the artifact in the data. As illustrated in FIG. 6A, by displaying histograms of the plural types of the comparison images, it is possible to provide the information used as the basis for the further detailed diagnostic support. FIG. 6B is a graph illustrating correlation of the Euclidean distance between the examination image and each of the four types of the comparison images. Here, while both of a vertical axis and a horizontal axis in FIG. 6B represent the Euclidean distance, the feature value space used to calculate the Euclidean distance differs therebetween. More specifically, the Euclidean distance on the feature value space determined by the feature value information, which is acquired as a result of performing the machine learning for a purpose of clarifying the distinction between benignancy and malignancy the most and is output by using the calculation unit, is set to the horizontal axis (the Euclidean distance (1)). The Euclidean distance on the feature value space determined by the feature value information, which is acquired as a result of performing the machine learning for a purpose of clarifying a distinction between a sum set of benignancy/malignancy and the other (no finding) and is output by using the calculation unit, is set to the vertical axis (the Euclidean distance (2)). Then, the virtual space image having such a horizontal axis and a vertical axis is generated. This is because, in the diagnosis of breast cancer using a breast imaging-reporting and data system (BI-RAD), possible benignancy (the probability of malignancy is 2% or less) is diagnosed in the category 3, and it is diagnosed that the probability of malignancy is 2 to 95% in the category 4. Thus, the image is insufficient to differentiate between benignancy and malignancy. However, there is a difficulty in using such label information as is for the machine learning. Accordingly, while the first machine learning is performed to derive the highest differentiation between benignancy and malignancy, it is effective that another machine learning is performed using the same image data such that more than the categories 1, 2 are differentiated. Here, the category 1 is negative, and the category 2 is positive. Just as described, the reliability of the determination result is improved when the information output by the plural feature value spaces is combined.

As it has been described so far, the virtual space image is generated only on the basis of the information such as the feature value space 50 illustrated in FIG. 4A. However, the virtual space image according to this embodiment is not limited to such an aspect. For example, an index, to which the person is used, is provided as a vertical axis, and, for example, the size of the feature area identified by the well-known image recognition technology or an average value of the gray scale value of the feature area is combined with the one-dimensional virtual area image 56 as illustrated in FIG. 4B. In this way, the two-dimensional image can be generated and displayed.

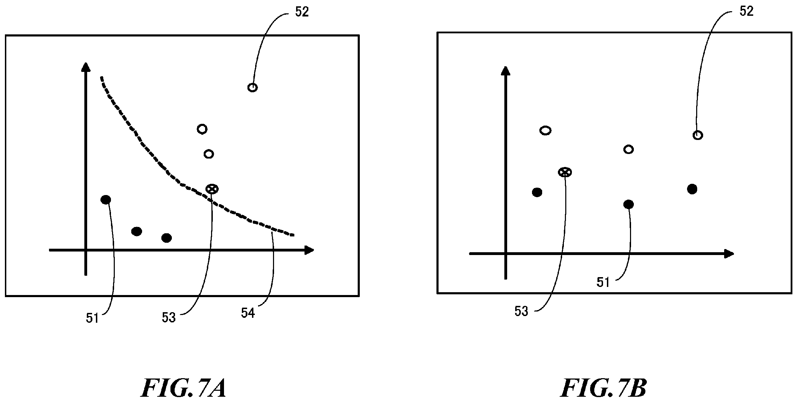

FIG. 7 includes schematic graphs in a case where the different virtual spaces are combined and displayed. The virtual space shown in FIG. 7A is the two-dimensional virtual space generated by using the N-dimensional parameter as the feature value information, and the examination image and the comparison images are plotted thereon. Meanwhile, the virtual space shown in FIG. 7B is the two-dimensional virtual space generated by using a diagnostic index or the like that has conventionally been used, and the examination image and the comparison images are also plotted thereon. The diagnostic index or the like described herein includes diagnosis data indicating a degree of approximation of appearance, a finding by the doctor or the like, and an index that is based on the basis of a diagnostic guideline. That is, it can be said that the virtual space shown in FIG. 7A is created on the basis of the feature value information calculated using the learned model. Meanwhile, it can be said that the virtual space shown in FIG. 7B is created on the basis of the feature value information that is familiar to a person such as the doctor.

Preferably, in order to facilitate understanding of relevance between these virtual spaces, the information in the virtual spaces is associated. As the association described herein, any of various types of processing can be adopted as long as processing identifies a relationship between the information displayed in the different virtual spaces. An example of such processing is processing to add the same visual effect such as commonalizing shapes and colors of points of the same comparison images plotted on these two virtual spaces. In this way, it is possible to supplement the diagnostic determination by the user with further another virtual space proposed for such a feature that cannot be separated in one of the virtual spaces.

FIG. 8 includes schematic views illustrating another example of the display in the display unit 16. In FIG. 8, the comparison images, the distance of each of which from the examination image is determined to be short on the basis of the calculated multidimensional parameter, are juxtaposed and displayed. In this example, the examination image and the similar case images of benignancy and malignancy are displayed together. For example, when the user specifies a particular area in the virtual space image 45 (see FIG. 3), the comparison image, a degree of similarity of which is high, in other words, the comparison image, the distance of which from the examination image is extracted from the comparison images that exist in such an area. As the comparison images of the similar case, the specified number of the malignant case image and the specified number of the benign case image that are the closest to the examination image are extracted from a specified range centered on the examination image in the feature value space 50. For example, the examination image 20 is displayed at the center of the display unit 16, and extracted benign case images 27 and extracted malignant case images 25 are respectively displayed according to the degree of similarity in a benign case image display area 26 and a malignant case image display area 24 provided on both sides. When the thus-displayed images are displayed in alignment, the degree of similarity therebetween can visually be acknowledged. The benign case images 27 herein may include, in addition to the comparison image including the benign tumor, the comparison image including the artifact and the comparison image not including the tumor or the like.

FIG. 8A shows a display example at the time when the malignant case image is not detected while the plural benign case images exist within the specified range centered on the examination image in the virtual space. In the benign case image display area 26, the three benign case images 27 are displayed. Meanwhile, the image displayed in the malignant case image display area 24 does not exist. In this case, it is obvious that there is no similar malignant case image. Accordingly, it is extremely obvious to the doctor that a possibility of the portion of the feature area 21 being the malignant tumor is low, and the diagnostic determination result by the diagnostic support system 1S leaves no doubt. FIG. 8C shows an example display at the time when only the malignant case images 25 are displayed as the similar cases in the malignant case image display area 24. Similarly, also in this case, the doctor can clearly determine that the feature area 21 is highly possibly the malignant tumor, and the diagnostic determination result by the diagnostic support system 1S leaves no doubt.

Meanwhile, FIG. 8B shows an example display of a case where the differentiation between benignancy/malignancy is vague on the feature value space 50. The three malignant case images 25 and the three benign case images 27 are displayed in the malignant case image display area 24 and the benign case image display area 26 of the examination image 20, respectively. The display case as shown in FIG. 8B is a case where it is impossible to clearly discriminate benignancy or malignancy. In such a case, it can be said that, instead of forcibly providing the determination result by the diagnostic support system 1S, this case is considered as a vague case, and the doctor preferably makes the determination. The doctor can select each of the case images and read the case information. In such a case, the doctor becomes conscious of vagueness of the determination of the examination image of the case and thus can easily make a determination such as biopsy of the tissue, follow-up, or the like.

As shown in FIG. 8, when the display unit 16 displays the malignant case images 25 and the benign case images 27, these selected images are preferably displayed in an ascending order according to the degree of the similarity, with values representing the similarity identified by the control unit 12, or with the visual effects according to the degree of the similarity such that the similarity of these selected images to the examination image can be understood at first glance.

Just as described, the images of two or more types such as properties of benignancy (normal) and malignancy are displayed as the comparison images in alignment. In this way, the doctor or the like can check the adequacy of the diagnosis by the machine. Instead of the user who designates the specified area in the virtual space image 45, the control unit 12 may select and display the comparison images, which are calculated in advance, juxtaposed, and displayed. In this case, the distance on the feature value space can be identified only from the feature value information. Thus, the virtual space image 45 does not always have to be displayed. Alternatively, the display mode shown in FIG. 8 or the display mode shown in FIG. 3 may be selected and appropriately changed by the user for the display.

<Second Embodiment

In the first embodiment that has been described so far, the description has been made on the example in which the comparison images constructed of the three-dimensional image data are displayed when the examination image and each of the comparison images are compared to derive the diagnostic determination. As another display example, when the comparison images are displayed, a drawing, such as a heat map, that has a visual effect to further prominently show the feature per pixel may be added to the lesion estimated area in each of the displayed images, and may be shown in each of the images. At this time, the image and the drawing such as the heat map may be superposed or juxtaposed with each other.

In regard to the comparison image, the location of the tumor and the lesion estimated area in the three-dimensional image are designated in advance, and information thereon is linked as the label information to the comparison image. Thus, the drawing such as the heat map can additionally be shown with reference to this label information. Meanwhile, the area to be checked is not identified in the examination image that is checked by the user. According to this embodiment, the user can efficiently identify the lesion estimated area to be checked from the three-dimensional examination image with reference to the lesion estimated area that is estimated by the machine learning technique, for example. Thus, it is possible to reduce a burden of the interpretation of the image.

In the case where the comparison image is of the normal tissues or the like, the lesion area is not shown. Meanwhile, an area that is estimated as a false lesion in the process of machine learning is shown. Thus, it is possible to reduce a chance of making a false-positive determination. This embodiment corresponds to an example in which the present invention is applied to computer-aided detection (CADe).

<Third Embodiment>

In the first embodiment and the second embodiment, the lesion estimated area is estimated by using the machine learning technique. Thereafter, the user determines the adequacy of the determination. In another embodiment, the user may first identify the lesion estimated area in the examination image. Thereafter, the comparison image having a similar area to the identified area may be presented.

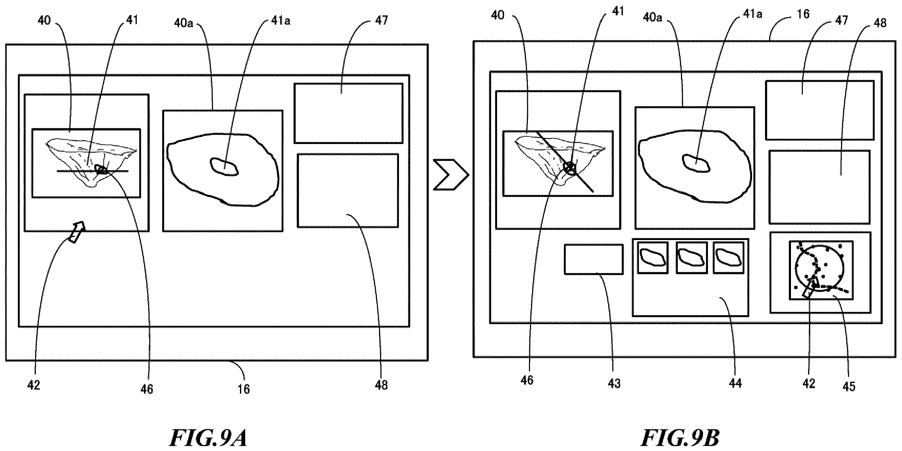

FIG. 9 includes schematic views illustrating display examples of the display in the display unit 16 in this embodiment. FIG. 9A shows the three-dimensional examination image 40 of the breast as the subject, which is acquired from the imaging device 4 through the communication control unit 11, on a right side of the display unit 16. In the examination image 40, there exists a feature area 41 with the high possibility as the tumor that should carefully be observed. At a center of the display unit 16, a cross section or an area (an examination image 40a, a feature area 41a) in the three-dimensional examination image is displayed. An angle and a position of the cross section, which is displayed as the examination image 40a, in the three-dimensional examination image 40 are indicated by using a line 49 that indicates a display cross-sectional position presented with respect to the three-dimensional examination image 40. The doctor or the like can change a position and an angle of this line 49 with a pointer 42 when operating a mouse (not shown) and can thereby select a specified cross section. The cross section displayed as this examination image 40a may automatically be determined by the control unit 12 so as to indicate an area with a high tumor existence probability.

Fig. 9B is an example display in which a diagnostic determination is made on the feature area 41a in the examination image 40a that is displayed when the pointer 42 is used to select the particular cross section for display. A determination result 43 is displayed at a lower right position of the display unit 16. For example, the determination result is an existence probability of the tumor, benignancy/malignancy differential determination, a corresponding diagnosis name, or the like. However, the determination result is not limited thereto. In addition, as the display method of the determination result 43, the method for using the specified visual effect such as highlighting the feature area 41a instead of the text information may be adopted. Furthermore, as an image showing the basis for the determination result, a virtual space image 45 is displayed in a lower right portion of the display unit 16, and the comparison images, which are designated by the doctor or the like using the pointer 42, for the virtual space image 45 are displayed as comparison images 44 showing the similar case in a lower center portion of the display unit 16.

According to this embodiment, the user can identify the area, the examination of which is desired, and extracts a similar case to the lesion area. In this way, it is possible to improve efficiency of such differential work by the user that identifies the type of the identified lesion. This embodiment corresponds to a case where the present invention is applied to a so-called computer-aided diagnosis (CADx).

All references, including publications, patent applications, and patents, cited herein are hereby incorporated by reference to the same extent as if each reference were individually and specifically indicated to be incorporated by reference and were set forth in its entirety herein.

The use of the terms "a" and "an" and "the" and similar referents in the context of describing the invention (especially in the context of the following claims) is to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

According to the diagnostic support system and the diagnostic support method of the present invention, as the basis for the display of the similar image as the determination result and the display of the probability that the biological information is included, the similarity between the examination image and each of the plural comparison images is provided to the user, which contributes to improvement in diagnosis efficiency.

DESCRIPTION OF REFERENCE NUMERALS

| | |
|---|---|
| 1S | Diagnostic support system |
| 2 | Diagnostic support device |
| 3 | Pre-acquired data storage unit |
| 4 | Imaging device |
| 11 | Communication control unit |
| 12 | Control unit |
| 13 | Calculation unit |
| 14 | Virtual space data storage unit |
| 15 | Display control unit |
| 16 | Display unit |
| 17 | Input unit |
| 18 | Speculation unit |
| 19 | Pre-processing unit |
| 20, 40, 40a, 53 | Examination image |
| 21, 41, 41a | Feature area |
| 24 | Malignancy-side display area |
| 25 | Malignant case image |
| 26 | Benignancy-side display area |
| 27 | Benignant case image |
| 42 | Pointer |
| 43 | Determination Result |
| 44 | Comparison image as similar case |
| 45 | Virtual space image |
| 46 | Lesion estimated area |
| 47 | Patient information display unit |
| 48 | Imaging condition display unit |
| 49 | Line |
| 50 | Feature value space |
| 51, 61 | Point corresponding to malignant case image |
| 52, 62 | Point corresponding to benign case image |
| 54 | Benignancy/malignancy discriminant line |
| 56 | One-dimensional virtual space image |
| 57 | Two-dimensional virtual space image |
| 63 | Point corresponding to artifact image |
| 64 | Point corresponding to normal tissue image |

**Claims**

1. A diagnostic support system comprising:

   a pre-acquired data storage unit for storing, as comparison images, two or more images of internal information of a subject, the two or more comparison images being acquired in advance and each comparison image including image data, label information indicating biological tissue information or shape information, and feature value information with which a degree of similarity among the two or more comparison images can be identified;
   a calculation unit for inputting image data of an examination image acquired from a subject as an examination target to a learned model that has learned the two or more comparison images, so as to calculate feature value information of the examination image; and
   a display unit for displaying the similarity between the examination image and each of the two or more comparison images on the basis of the feature value information of the two or more comparison images and the feature value information of the examination image.

2. The diagnostic support system according to claim 1, wherein the feature value information is information including a multidimensional parameter, and
   further comprising a control unit for identifying, as a value indicating the similarity, a distance between the examination image and each of the two or more comparison images on a feature value space that has the multidimensional parameter constituting the feature value information as a coordinate.

3. The diagnostic support system according to claim 1, wherein the feature value information is information including a multidimensional parameter,
   further comprising a virtual space data storage unit for generating a first virtual space by using the feature value information, the first virtual space including a displayable dimensional number and being used to visually recognize the similarity, and
   wherein, in addition to the two or more comparison images, the display unit displays a first virtual space image, the examination image being plotted on the first virtual space in the first virtual space image.

4. The diagnostic support system according to claim 1, wherein the feature value information is information including linguistic expression corresponding to the two or more comparison images and the examination image,
   further comprising a virtual space data storage unit for generating a second virtual space by using the feature value information, the second virtual space being used to visually recognize the similarity, and
   wherein, in addition to the two or more comparison images, the display unit displays a second virtual space image, the examination image being plotted on the second virtual space in the second virtual space image.

5. The diagnostic support system according to claim 3 or claim 4, wherein the similarity is identified on the basis of a distance between the examination image and each of the two or more comparison images on the virtual space image.

6. The diagnostic support system according to any one of claims 1 to 5, wherein the display unit displays one or more of the two or more comparison images, the similarity of which to the examination image is high.

7. The diagnostic support system according to any one of claims 1 to 6, wherein the display unit adds a specified visual effect to display of the two or more comparison images on the basis of the label information.

8. The diagnostic support system according to any one of claims 1 to 7, further comprising a speculation unit for speculating on the basis of the examination image and the two or more comparison images whether preset biological information is included in the examination image.

9. The diagnostic support system according to any one of claims 1 to 8, further comprising a pre-processing unit for adjusting a format of the image data of the examination image to a format with which the calculation unit can calculate the feature value information.

10. A diagnostic support method using a computer, comprising:

    storing, as comparison images, two or more images of internal information of a subject, the two or more comparison images being acquired in advance and each comparison image comprising image data and feature value information with which a degree of similarity among the two or more comparison images can be identified;

inputting image data of an examination image acquired from a subject as an examination target to a learned model that has learned the two or more comparison images, so as to calculate feature value information of the examination image; and

displaying the similarity between the examination image and each of the two or more images on the basis of the feature value information of each of the two or more images and the feature value information of the examination value.

EP 3 832 663 A1

DIAGNOSTIC SUPPORT SYSTEM 1S

PRE-ACQUIRED DATA STORAGE UNIT — 3

IMAGING DEVICE 4

31

SP

B

T

TRANSCEIVING CONTROL UNIT 32

STORAGE UNIT 35

IMAGE GENERATION UNIT 33

DEVICE CONTROL UNIT 34

DIAGNOSTIC SUPPORT DEVICE 2

COMMUNICATION CONTROL UNIT 11

CONTROL UNIT 12

CALCULATION UNIT 13

VIRTUAL SPACE DATA STORAGE UNIT 14

PRE-PROCESSING UNIT 19

SPECULATION UNIT 18

INPUT UNIT 17

DISPLAY UNIT 16

DISPLAY CONTROL UNIT 15

FIG. 1

START

STORE COMPARISON IMAGES — S1000

GENERATE MULTIDIMENSIONAL
PARAMETER OF COMPARISON IMAGE — S1100

ACQUIRE EXAMINATION IMAGE — S1200

ACQUIRE COMPARISON IMAGE
AND MULTIDIMENSIONAL PARAMETER — S1300

CALCULATE MULTIDIMENSIONAL
PARAMETER OF EXAMINATION IMAGE — S1400

GENERATE AND DISPLAY
VIRTUAL SPACE IMAGE — S1500

DISPLAY COMPARISON IMAGE — S1600

END

*FIG.2*

**FIG.3A**

**FIG.3B**

*FIG.4B*

*FIG.4A*

EP 3 832 663 A1

FIG.5A

FIG.5B

FIG.5C

FIG.5D

NUMBER OF DATA

EUCLIDEAN DISTANCE "FROM EXAMINATION IMAGE TO COMPARISON IMAGE" ON VIRTUAL SPACE

51

52

53

24

*FIG6B*

*FIG.6A*

**FIG.7B**

**FIG.7A**

*FIG.8C*

*FIG.8B*

*FIG.8A*

*FIG.9A*

*FIG.9B*

EP 3 832 663 A1

<div align="center">**INTERNATIONAL SEARCH REPORT**</div>

| | International application No. |
|---|---|
| | PCT/JP2019/030091 |

**A. CLASSIFICATION OF SUBJECT MATTER**
Int.Cl. G16H50/20(2018.01)i, A61B5/00(2006.01)i

According to International Patent Classification (IPC) or to both national classification and IPC

**B. FIELDS SEARCHED**

Minimum documentation searched (classification system followed by classification symbols)
Int.Cl. G16H50/20, A61B5/00, G06Q10/00-99/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y<br>A | JP 2011-118543 A (SHIZUOKA-KEN) 16 June 2011, paragraphs [0031], [0033], [0036], [0041], [0042], [0048], [0053] (Family: none) | 1,2,6-10<br>3-5 |
| Y<br>A | MARYWKKWN, L. Giger, 4. AIを用いた乳房 CADx の開発における進歩, INNERVISION, 25 June 2018, vol. 33, no. 7, pp. 13-17, ("4. Advances in the development of Breast CADx with AI") | 1,2,6-10<br>3-5 |

☒ Further documents are listed in the continuation of Box C.    ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 10 October 2019 (10.10.2019) | 21 October 2019 (21.10.2019) |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
|---|---|

Form PCT/ISA/210 (second sheet) (January 2015)

**INTERNATIONAL SEARCH REPORT**

International application No.

PCT/JP2019/030091

C (Continuation).    DOCUMENTS CONSIDERED TO BE RELEVANT

| Category* | Citation of document, with indication, where appropriate, of the relevant passages | Relevant to claim No. |
|---|---|---|
| Y<br>A | WO 2014/024453 A1 (KONICA MINOLTA, INC.) 13 February 2014, paragraph [0137], fig. 16A, B, C & US 2015/0196281 A1, paragraph [0164], fig. 16A, B, C | 1,2,6-10<br>3-5 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011118543 A
- JP 2016045662 A
- JP 2018144549 A
- WO 2017051903 A